(19) Europäisches Patentamt
European Patent Office
Office européen des brevets

(11) EP 2 023 816 B1

(12) **EUROPEAN PATENT SPECIFICATION**

(45) Date of publication and mention
of the grant of the patent:
**12.03.2014 Bulletin 2014/11**

(21) Application number: **07733699.8**

(22) Date of filing: **21.05.2007**

(51) Int Cl.:
***A61B 5/103*** (2006.01)

(86) International application number:
**PCT/GB2007/050278**

(87) International publication number:
**WO 2007/135462 (29.11.2007 Gazette 2007/48)**

(54) **BALANCE MONITOR**

GLEICHGEWICHTSMONITOR

SYSTEME DE CONTROLE DE L'EQUILIBRE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **19.05.2006 GB 0609999**

(43) Date of publication of application:
**18.02.2009 Bulletin 2009/08**

(73) Proprietor: **Teesside University
Middlesborough
Cleveland TS1 3BA (GB)**

(72) Inventors:
• SPEARS, Iain R
  **Middlesborough TS7 8AN (GB)**
• QUINN, Gary
  **Middlesborough TS12 2DN (GB)**

(74) Representative: **Killough, Kieran Paul et al
Harrison Goddard Foote LLP
4th Floor, Merchant Exchange
17-19 Whitworth Street West
Manchester M1 5WG (GB)**

(56) References cited:
**WO-A-99/44502        US-A- 4 631 676
US-A1- 2004 002 642    US-A1- 2005 105 772**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a device and method for monitoring balance, in particular but not exclusively in connection with rehabilitation of patients recovering from strokes or elderly patients, and also in connection with sports motion analysis. More particularly, the invention relates to an optical balance monitor.

BACKGROUND

Rehabilitation of human patients following a stroke or the like can be a lengthy process, and monitoring progress is of great importance to doctors and patients alike. Strokes can affect people in different ways, depending on the type of stroke, the area of the brain affected and the extent of the brain injury. Brain injury from a stroke can affect the senses, motor activity, speech and the ability to understand speech. It can also affect behavioural and thought patterns, memory and emotions. Paralysis or weakness on one side of the body is common. Accordingly, monitoring a patient's balance, for example when walking, and in particular monitoring improvement of such balance over time, can be a useful indication of rehabilitation. Monitoring of balance is also relevant in assessing elderly patients.

When a subject is maintaining balance in an upright position, small body oscillations in the anterior-posterior and medial-lateral directions inevitably arise. The magnitude of these oscillations may be referred to as the amount of postural sway. The amount of postural sway is considered indicative of a person's neuro-muscular well-being. The measurement of sway is of considerable interest from practitioners and researchers in a range of health sciences including toxicology, physiotherapy and sport science.

Typically, a doctor or other appropriate healthcare professional can make a subjective assessment of balance by observing a patient's gait, or by asking a patient to perform particular movements. However, such subjective assessments are by necessity limited in their usefulness, especially if the patient in question is assessed by different doctors during the course of recovery.

A range of types of system have previously been developed to provide quantitative data during static posturography trials.

In the case of static posturography trials, the systems can be broadly distinguished as those measuring the kinetics of movement by measuring forces, and those measuring the kinematics of movement by measuring actual movements.

Force measurement plate devices measure a distribution of force applied to a plate of the device and allow the position of the instantaneous centre of pressure (COP) to be quantified. Such devices provide an output corresponding to the location of the COP in Cartesian coordinates with respect to axes of the device. Force measurement plate devices have become a very popular tool for research into the kinetics of balance. In contrast, measurement of the kinematics of sway has made use of techniques ranging from electromagnetic motion sensors to optical motion capture. These latter tools have been used to predict the position of the centre of mass (COM), the centre of mass being the controlled variable during upright standing. A disadvantage of these systems is their lack of portability and high cost. Consequently they are used for clinical assessments of balance only in specialist clinics.

In the case of dynamic posturography trials, it is known, for example from US 4,631,676 and DE 4328371, to provide systems for recording and analysing body movements and gait, in particular for analysing athletic performance and rehabilitation after surgery. These systems generally involve placing a plurality of reflective or illuminated markers on a subject, for example along a subject's leg, and then using one or more specially adapted video cameras to record the movement of the markers as the subject walks along a predetermined path or on a treadmill. These systems are relatively complex, in that several specialised video cameras are typically required, and in that special care is needed when placing the markers on the subject so as to define exactly the movement that is being measured.

Even more complex systems are known, for example from US 6,324,296, which seek to provide a full analysis and recordal of human gait and movement with a view to use in computer graphics animation techniques. Here, a large number of illuminated markers are disposed all over a subject so as to allow the movement of each part of the subject to be captured, and several image capture devices are employed so as to capture a full range of movement and to ensure proper triangulation of every point in three-dimensional space. The illuminated markers may be made to flicker at predetermined and mutually distinguishable frequencies so as to allow points in the recorded images to be identified and distinguished.

US-2005/0105772 (Voronka et al.) describes an optical system that tracks the motion of objects, including human body or portions thereof using a plurality of three-dimensional active markers based upon triangulation from data read via multiple linear CCDs through cylindrical lenses. Each marker is lit in sequence so that it is in sync with a frame capture using the imaging system positioned and oriented so as to provide a basis for computing three-dimensional location.

WO-A-99/44502 (Claussen et al.) describes a system for evaluating a head and trunk movement pattern of a subject by configuring a plurality of markers to move together with the body of a subject. The locus curve of each marker is detected in a three dimensional area (using two cameras) as a function of time and is stored as a data field of a data record that is common to all markers. Computer assisted analysis of the locus curves is then carried out by means of a computer whereby a characteristic is determined and evaluated in terms of pattern recognition by comparing it to the corresponding reference values.

These known systems tend to be complex and relatively expensive, and are often over-complicated for some simple applications. In particular, objectively assessing balance can be a costly process for health care professionals, since the prior art motion capture systems are expensive and not portable, and are therefore found only in specialist clinics.

BRIEF SUMMARY OF THE DISCLOSURE

In a first aspect of the present invention there is provided a system for monitoring a person's balance, the system comprising:

(i) a unit bearing at least one indicium, wherein the at least one indicium is/are configured to provide at least two spatially resolved reference positions, the at least one indicium having a fixed predetermined spatial configuration on the unit; and
ii) an image capture device having a single field of view;

wherein either the unit or the image capture device is provided with a means for attachment to a person such that the unit or device is locatable generally at a centre of balance of a subject, the system being configured to record movement, in the single field of view of the image capture device, of the at least one indicia with reference to the subject's centre of balance and calculate the distances of the at least two spatially resolved reference positions of the unit from the image capture device so as to obtain an objective measure of balance.

In the context of the present application, a person's centre of balance may be considered to be equivalent to the person's centre of mass.

The system has the advantage that it may be constructed and operated in a less costly and more simpler manner than prior art systems. Furthermore, embodiments of the system are more reliable and less costly to maintain than prior art systems.

A system according to embodiments of the invention may be assembled with relatively inexpensive and readily available components such as a webcam and an array of light emitting diodes.

In the case that a plurality of indicia are present, individual indica may be identified and differentiated from each other in the captured image by making use of different light characteristics of the indicia, be these colour or shape or whichever characteristic is chosen.

By analysing changes in the relative spacing in the captured image of the indicia, it will be clear that an analysis of relative movement of the actual indicia with reference to a notional or real centre of balance of a patient during the course of a static or dynamic posturography assessment can be determined, and hence an objective measure of a patient's balance can be obtained.

It will further be understood that the unit bearing the indicia may, instead of being attached to the patient, be mounted on a stationary stand or the like, and an image capture device be attached to the patient at or near his/her centre of balance. Similar relative movements will take place and be measured during assessment.

Preferably the system further comprises a computer in communication with the image capture device.

This feature enables data acquired by the image capture device to be processed according to algorithms associated with the system.

Preferably the at least one indicium is/are configured to provide at least two spatially resolved reference positions on the unit.

The at least two spatially resolved reference positions may be features of an image provided on the unit such as features of a face or other feature of a cartoon character or other image.

In use, the unit may be fitted to a subject (such as a patient) and an image capture device (e.g. a video camera, webcam or even a mobile telephone camera) set up in a preferably stationary configuration so as to face the patient and the device.

The patient may then be asked to stand (eyes closed, eyes open, on foam, on rigid floor) without moving his/her feet so as to obtain a static posturography assessment. Alternatively or in addition, the patient may be asked to walk towards the image capture device (or walk on a treadmill facing the image capture device) so as to obtain a dynamic posturography assessment or gait analysis.

A minimum of two spatially resolved features (such as light emitting devices or features of a cartoon character's face) are required for a meaningful assessment of balance by analysis of images obtained by the image capture device. By measuring the distance between (say) two light emitting devices at a known position of the patient relative to the image capture device, and also knowing the relative positions of the light emitting devices and the patient's centre of balance, it is possible to calculate a distance of the unit from the image capture device at any given time, as well as to calculate a degree of side-to-side (medial-lateral) and front-to-back (anterior-posterior) movement of the patient about his/her centre of balance.

This eliminates the need for an operator of the system to measure the distance manually and input the distance to the system.

Preferably the unit is arranged to bear at least two light emitting or reflecting indicia, the indicia having a fixed predetermined spatial configuration on the unit.

This feature has the advantage that the indicia may be more readily detected by the computer.

Preferably at least one of the indicia is arranged to emit/reflect light of a different characteristic from the other indicium/indicia.

This feature has the advantage that indicia may be more readily and conveniently distinguished from one another in an acquired image.

In the case that one indicium is not distinguishable from another indicium by means of its appearance in a captured image, but only by means of its position relative to another indicium, its relative position may be used to identify the position of the indicium on the unit. Alternatively or in addition, real time tracking of the position of one or more indicia may be performed in order to enable the computer to continually identify each indicium. Thus, if one or more indicia move out of the field of view, it is possible to continue to identify the location on the unit of each indicium that remains visible in an acquired image.

Preferably the unit is provided with two light emitting/reflecting indicia, each indicium emitting/reflecting light of a different characteristic from the other indicia/indicium, the indicia having a fixed predetermined spatial configuration.

The light emitting/reflecting indicia may be configured to project images having different shapes.

This feature enables individual indicia to be more easily identified when a single-colour capture device is used, such as a black and white capture device.

The unit may comprise three light emitting /reflecting indicia. The three light emitting/reflecting indicia may have a linear arrangement.

The unit may comprise three light emitting/reflecting indicia, respectively emitting/reflecting red, green and blue light to correspond with red, green and blue channels of an image capture device.

Preferably a central light emitting/reflecting indicium is locatable generally at the centre of balance of a subject.

It will be appreciated that reference to location of an indicum at the centre of balance of a subject includes location of an indicium at a location close to the centre of balance. The centre of balance of a subject may of course be a location within a body of a subject. Thus, reference to location of a centre of balance of a subject includes location forward of the centre of balance along a line passing through the centre of balance and parallel to an anterior-posterior axis of the subject.

This allows for easier calibration and processing of measurements, since the centre of balance does not need to be inferred in each image, but is directly indicated by one of the light emitting devices.

Preferably the unit comprises five light emitting/ reflecting indicia.

This feature has the advantage that an orientation of the unit with respect to the capture device and a distance of the capture device from the unit may be more easily determined.

Preferably the five light emitting/reflecting indicia are arranged such that a central light emitting/reflecting indicium is surrounded by four light emitting/reflecting indicia arranged so as to define corners of a notional quadrilateral enclosing the central light emitting/reflecting indicium.

In this arrangement, by measuring the relative spatial positions of and distances between the five light emitting devices, it is even easier to determine medial-lateral and anterior-posterior movement or tipping.

Preferably the four light emitting/reflecting indicia arranged to define corners are different in colour to the central light emitting/reflecting indicium.

This feature has the advantage that it is easier to distinguish the central indicium from the corner (or peripheral) indicia.

Preferably at least one of the four light emitting/ reflecting indicia arranged to define corners is of a different colour to the other three indicia arranged to define corners.

This feature has the advantage that it is easier to identify positions on the unit of one or more of the peripheral indicia.

Preferably the unit comprise at least one light-reflective indicium and the system further comprises a light source arranged to illuminate the at least one light-reflective indicium.

This feature has the advantage that the unit need not be an 'active' unit in the sense that the unit requires a power source (such as a battery) to power light emitting devices. Rather, devices requiring a power source may be made separate from the unit, thereby not being required to be worn by the subject. This has the advantage of reducing a weight of the unit. A unit having excessive weight may result in measurements that are not representative of a subject's true sway variable(s).

Preferably the light source is co-located with the image capture device.

This feature has the advantage that it simplifies construction of the system. In some embodiments of the invention the light source (whether a visible light source, an infra-red light source or a UV light source) is combined within a housing of the capture device. Thus, it is not necessary to provide a separately mounted light source. In some embodiments of the invention, ambient lighting provides sufficient illumination of the indicium/indicia for the system to operate in an effective manner.

Preferably the light source is an infra-red (IR) light source, said at least one light-reflective indicium being arranged to reflect IR light.

Preferably the image capture device is configured so as to capture images in which substantially all background detail is obscured, leaving only images of the at least one indicium.

This has the advantage that determination by the computer of the location of one or more indicium in a captured image is made easier.

Preferably the unit is provided with means for attaching the unit to a person such that the unit is locatable generally at a centre of balance of the person.

This feature has the advantage that the position of the unit more accurately tracks movement of the subject. In contrast, in the case that the unit is held in the hand of a subject, measurement of movement of the hand of the subject will result which does not necessarily provide an accurate measure of an amount of sway of the centre of balance/mass of the subject, even if the subject attempts to hold their hand still.

It will be appreciated however that embodiments of the invention may be used to measure movement (such as sway movement) of portions of a body of a subject such as one or more hands of a subject, or one or more feet of a subject. Thus, a subject may be required to hold the unit stationary using their hands or their feet, whilst images of movement of the hands or feet are captured and analysed.

Preferably the means for attaching the unit comprises a belt or strap or clip.

Preferably the system is further configured to determine a sway variable of the subject based on the measured movement of the unit.

Knowledge by a clinical practitioner of one or more sway variables associated with a subject is invaluable in determining an ability of the subject to stand still, relative to like subjects of similar age or other characteristic. Thus, an assessment of a condition of a subject may be made using data obtained using embodiments of the present invention.

Furthermore, knowledge of one or more sway variables by a clinical practitioner is invaluable in monitoring an improvement or deterioration in the ability of a subject to stand still. This may be facilitated for example by performing regular measurements of one or more sway variables of a subject over a period of time.

In a second aspect of the invention there is provided a method of monitoring a person's balance according to claim 15.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

BRIEF DESCRIPTION OF THE DRAWINGS

For a better understanding of the present invention and to show how it may be carried into effect, reference shall now be made by way of example to the accompanying drawings, in which:

FIGURE 1 shows an embodiment of an optical balance monitor unit according to the present invention;

FIGURE 2 shows a person wearing the unit of Figure 1, and facing an image capture device;

FIGURE 3 shows an image captured by the image capture device;

FIGURE 4 is a schematic illustration of a user-attachable portion of an optical balance monitor system according to a second embodiment of the invention;

FIGURE 5 is a schematic illustration of an optical balance monitor system according to the second embodiment;

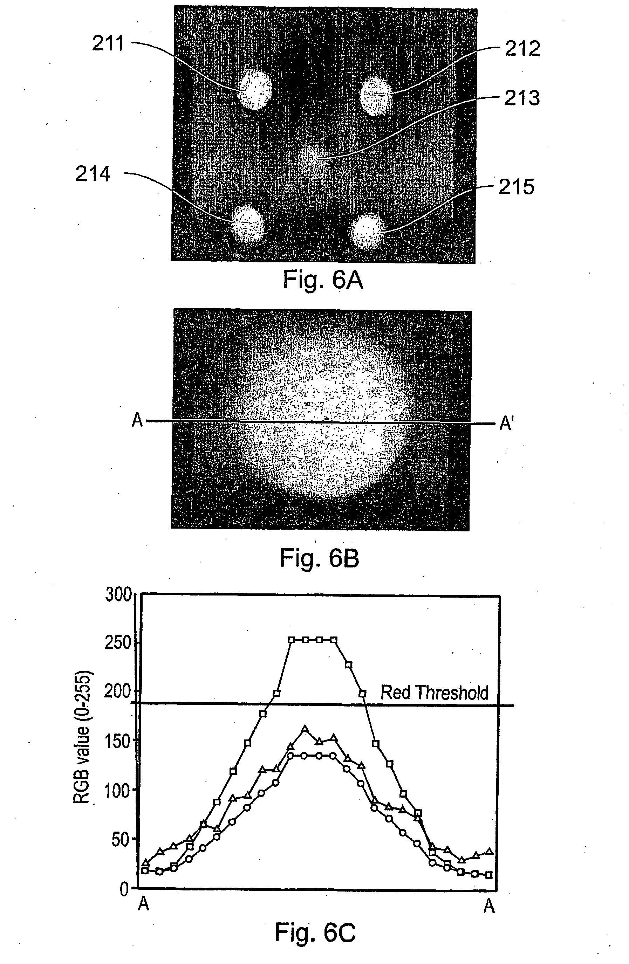

FIGURE 6 shows (a) an image captured by an image capture device in a system according to the second embodiment of the invention; (b) an enlarged image of a red LED taken from the image of (a); and (c) a plot of the intensity of red, green and blue light emitted by the LED;

FIGURE 7 is a schematic illustration of an image of five LED elements acquired by a system according to the second embodiment;

FIGURE 8 is a schematic illustration of respective positions of LED elements of an optical balance monitor system according to the second embodiment;

FIGURE 9 is a schematic illustration of a side view of a portion of an optical balance monitor system according to the second embodiment;

FIGURE 10 is a schematic illustration of respective tilt positions of a user-attachable portion of an optical balance monitor system according to the second embodiment;

FIGURE 11 shows a graph of the position of an LED element as a function of time as measured by an optical balance monitor system according to the second embodiment;

FIGURE 12 shows values of the coefficient of variation of measurements of (i) COP using a force-balance monitor (white bars); (ii) sway length using an optical balance monitor system according to the second embodiment (black bars); and (iii) COP calculated from the values of sway length obtained from the optical balance monitor system (grey bars) for subjects with (a) their eyes open and (b) their eyes closed.

FIGURE 13 is a plot of data values obtained from the optical balance monitor system (labelled 'B') and the force-balance monitor (labelled 'F') as a function of time over a 30s period;

FIGURE 14 shows a representative time-series of measurements of the position of the centre of pressure (COP) of a subject as determined by a force platform device (labelled 'F'), together with measurements of COP as calculated from COM data obtained by means of an optical balance monitor (labelled 'B');

Figure 15 shows a table of values of paired two-tailed t-tests for trials in the eyes open condition versus trials in the eyes closed condition for a system according to the second embodiment;

FIGURE 16 shows a unit (or housing) according to an embodiment of the invention; and

FIGURE 17 shows a system according to a third embodiment of the invention.

## DETAILED DESCRIPTION

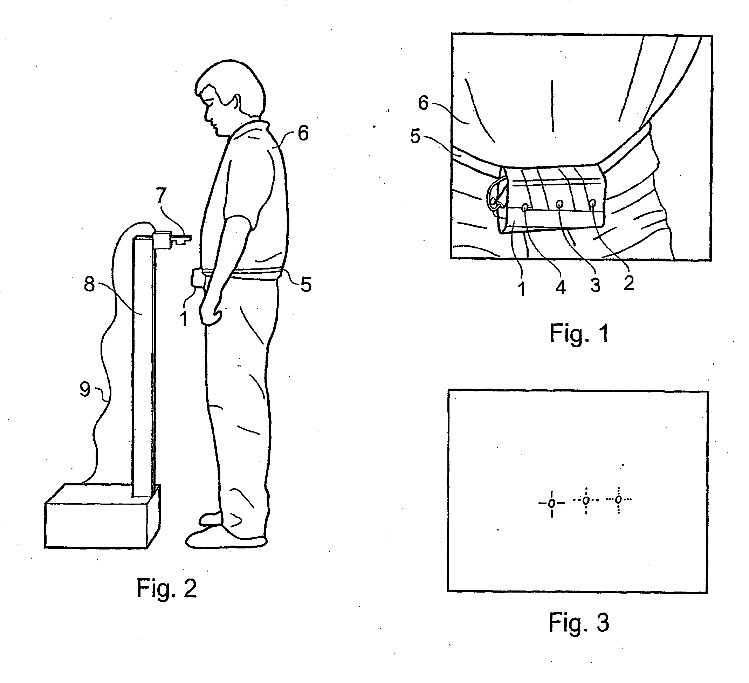

According to a first embodiment of the invention, a balance monitoring system comprises a housing 1 provided with a linear array of LEDs 2, 3, 4, which respectively emit blue, red and green light (Figure 1). The housing 1 is further provided with a belt 5 for fitting about the waist of a person 6, such that the red LED 3 is located generally at the person's centre of balance. The distance between the blue 2 and green 4 LEDs is fixed and known.

Figure 2 shows the person 6 wearing the housing 1, and a webcam or similar image capture device 7 mounted on a stand 8. The webcam 7 is connected to a computer (not shown) by cable 9.

In use, the person 6, wearing the housing 1, walks towards the webcam 7.

The webcam 7 records the image shown in Figure 3, which indicates the relative positions of the blue 2, red 3 and green 4 LEDs.

Because the absolute distance between the blue 2 and green 4 LEDs is known, it is possible to determine the absolute movement of the red LED 3 about a notional centre of balance no matter how far the housing 1 is from the webcam 7, even taking into account changes in this distance during image capture as the person 6 walks towards the webcam 7.

In a specific example, using a webcam 7 at a resolution of 320x240 (to allow the frame rate to be kept high) to look at an area of 320cm by 240cm, then each pixel will see a 1cm square, which is quite poor. However, by making the blue 2 and green 4 LEDs 8cm apart, and bearing in mind that the camera needs to see only a small area for balance monitoring, the small pixel resolution is adequate for this application.

Nevertheless, currently available webcams 7 and computers can record at 640x480 pixels without sacrificing the frame rate significantly.

The webcam 7 is configured to provide three "bit planes" of colour information - red, green, and blue. Each bit plane is a two dimensional array of 8 bit colour information (values range from 0 for the lowest intensity [no light] to 255 for the highest intensity light). Accordingly, the red LED 3 should only appear in the red bit plane. In practice, LEDs rarely transmit just their apparent colour, so red LEDs usually appear as red with a small amount of green and/or blue - but at too low a level to register in the human eye. Likewise with green and blue LEDs. Yellow is made up from green and blue, so a yellow LED should appear in both green and blue bit planes, but not in the red bit plane.

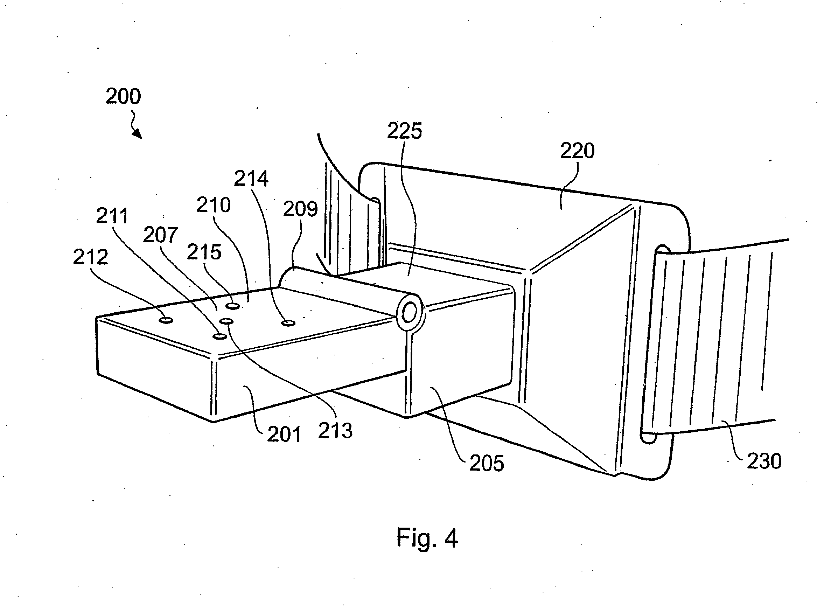

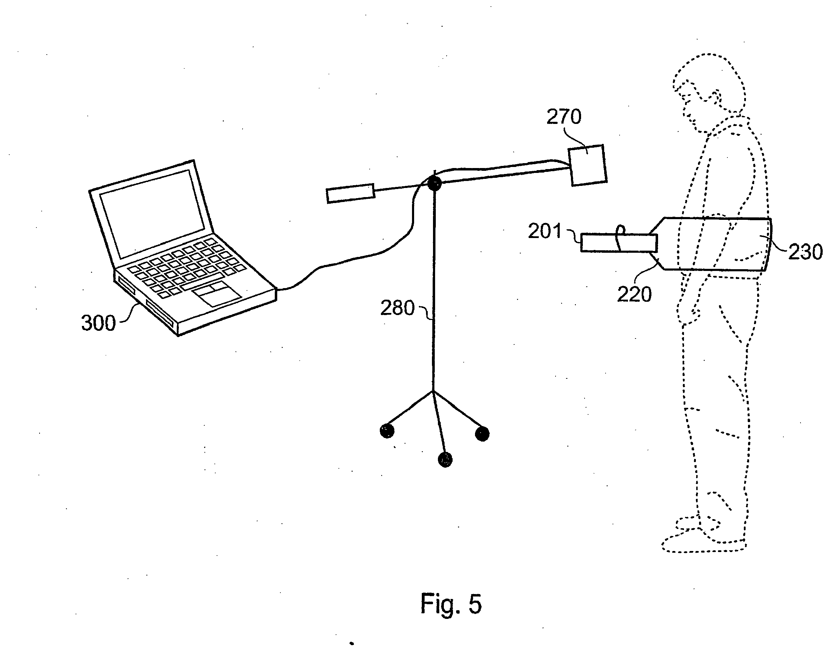

According to a second embodiment of the invention, an optical balance monitor system is provided as shown generally in Figures 4 and 5. A system according to the second embodiment has a user-attachable portion 200 having a housing 201 that is releasably fastenable to a holder 220 that is connected to a belt strap 230. The holder 220 has a recess 225 into which a portion of the housing 201 may be inserted. According to the second embodiment the housing 201 is fastenable to the holder 220 by means of a snap-fit connection. In alternative embodiments, the housing 201 is fastenable to the holder 220 by means of a friction fit, by means of a fastening element such as a screw or a bolt, by means of a hook and loop faster such as Velcro™ or any other suitable fastening arrangement.

The housing 201 has a base portion 205 and a LED-bearing flap portion 207. The flap portion 207 is coupled to the base portion 205 by means of a hinge member 209.

The flap portion 207 has a display face 210 that is provided with an array of five LED elements 211, 212, 213, 214, 215. The LED elements are arranged in the shape of the 'five' face of a gaming die. LED elements at corners of the array, indicated at 211, 212, 214 and 215 are of a red colour, whilst the LED element at the centre of the array, indicated at 213, is of a blue colour.

It will be appreciated that in alternative embodiments of the invention the shape of the array is a different shape. Furthermore, it will be appreciated that in some embodiments of the invention a different arrangement or combination of colours is employed. In some embodiments LED elements of the same colour only are used.

The flap portion 207 may be folded into juxtaposition with the base portion 205 for storage of the housing 201. In a folded configuration, display face 210 is in mutually confronting relationship with the base portion 205 of the housing 201. Thus, in the folded configuration, the LED elements are protected from damage, for example during storage or transport.

In an in-use configuration, flap portion 207 is arranged to project away from the base portion 205 of the housing 201. With the holder 220 attached to a user's waist, the display face 210 is configured to be superiorly directed. In other words, the display face 210 is configured to face in a generally upwards direction relative to the wearer.

The optical balance monitor system also has a camera module 270 (Figure 5), mounted on a stable support 280. The camera module 270 is positioned above the housing 201 in normal use, and is inferiorly directed towards the housing 201.

The camera module is connected to a processing device 300 in the form of a PC running a software program. The processing device 300 is configured to acquire images from the camera module 270 and to process the images in order to track movement of the LED elements of the user attachable portion 200. According to the second embodiment, the processing device 300 acquires a new image from the camera module 270 once every 33ms.

The processing device 300 is configured to determine the position in the acquired images of the centroid of each of the LED elements. The processing device 300 is thereby able to 'track' the position of each LED element with respect to the other LED elements. By tracking the position of each LED element with respect to the other LED elements, the system is able to determine the distance of the housing 201 from the camera module 270. The system is also able to determine the distance of the central blue LED element 213 from the camera module 270, and the distance moved by the blue LED element 213 with respect to edges of an acquired image.

In some embodiments of the invention the processing device 300 is also configured to calculate angles of rotation of the housing 201 with respect to the camera module 270 about an anterior-posterior axis, medial-lateral and superior-inferior axis. These angles of rotation are calculated with reference to the positions of LED elements in an acquired image, and a knowledge of distances between the LED elements.

It will be appreciated that in some embodiments of the invention, a different arrangement of LED elements, including the addition of further LED elements in further embodiments, may be used in order to determine accurately an angle of tilt of the housing 201.

It will be further appreciated that one or more indicia may be provided on the housing 201 in order to enable the optical balance monitor system to determine the position of the housing 201. In some embodiments of the invention, the housing is provided with an array of markings instead of LED elements in order to determine movement and tilt of the housing 201. In some embodiments the LED elements of the second embodiment are replaced by corresponding colour dots or circles. In further embodiments of the invention, the LED elements are replaced by shape. In some embodiments the shape is a square; in other embodiments the shape is a triangle, and in still further embodiments the shape is another quadrilateral. In some embodiments LED elements are replaced by a single or multiple shapes that correspond to an image such as of a cartoon character, or an object such as an animal or a car.

As in the case of the first embodiment, the camera module 270 of the second embodiment is configured to provide three bit planes of colour information corresponding to the colours red, green and blue, respectively.

The processing device 300 is configured to determine the centroid of each of the red LED elements by applying a threshold level to the red bit plane signal received by the processing device 300 from the camera module 270. A suitable threshold level for the red signal is shown in the graph of Figure 5(b).

The determination of the centroid of an LED involves scanning the image and checking each pixel for three conditions. The three conditions are (i) whether the local red value of the pixel (in other words, the value of the red bit plane signal received from the camera module 270) is greater than the threshold level; (ii) whether the local red value is greater than the local blue value; and (iii) whether the local red value is greater than the local green value. If a pixel meets these criteria, its coordinates are stored in a memory of the processing device 300.

Once an entire image has been scanned, the coordinates of the centroids are calculated as the mean x and y positions of all the red pixels associated with a given red LED element. A sub-pixel level of accuracy in determining the position of the centroid of an LED element in an acquired image is thereby introduced.

The process is performed for each of the four red LED elements. A similar process is performed in respect of the blue LED element 213 by determining whether the local blue value of a pixel is greater than a blue bit plane signal threshold level, and whether the local blue value is greater than the local red and green values.

In some embodiments the blue bit plane signal threshold level is set to a different value with respect to the red bit plane signal threshold level in order to obtain optimum results.

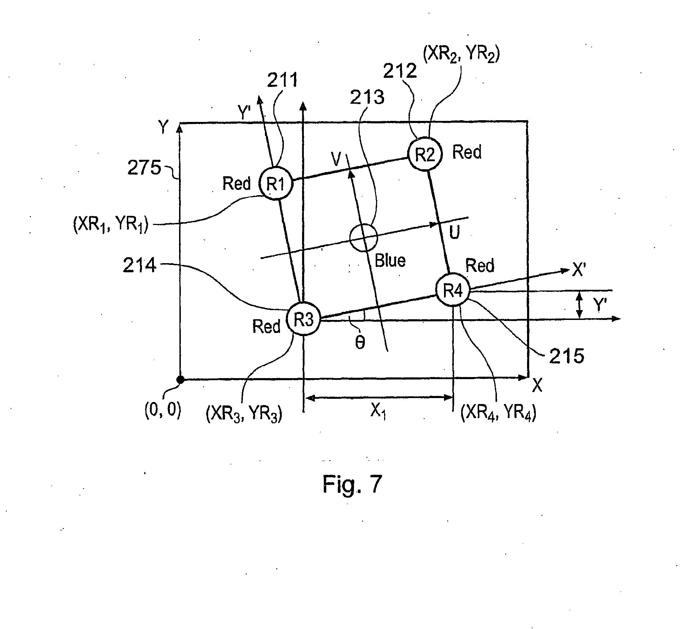

The processing device 300 is further configured to compare the coordinates of the blue LED with the intersection of the diagonals joining the red LEDs. For example, with respect to the position of the wearer of the user-attachable portion 200, a calculation is made of the intersection of the diagonals joining the top-left and bottom-right LED elements (elements labelled 211 and 215, Figure 7) and the top-right and bottom-left LED elements (elements labelled 212 and 214). If the intersection does not fall within a predetermined tolerance (e.g. a tolerance of 0.01 mm), the threshold levels are modified.

According to the second embodiment the red threshold level for all red LED elements is the same. The red and blue threshold levels start at a value of 1 and are incremented in steps of 1 unit in the range 0-255 until the required tolerance is reached.

Once a determination of the positions of the four peripheral red LED elements (the LED elements labelled 211, 212, 214, 215) in an acquired image has been performed, as described above, the image can be calibrated in terms of real space coordinates.

Figure 7 is a schematic illustration of an image acquired using the camera module 270. A boundary of the image is indicated at 275, and an origin of the image indicated (0,0). An X-axis and a Y-axis are labelled. Positions of the red LED elements are defined in Cartesian coordinates with reference to axes X, Y and origin (0, 0). These positions are those determined as described above.

With reference to Figure 7, the four peripheral red LED elements 211, 212, 214, 215 are labelled R1, R2, R3 and R4, respectively.

The coordinates of R1 are written (XR1, YR1) and the coordinates of R2 are written (XR2, YR2), and similarly for R3 and R4.

The processing device 300 is configured to calculate the distance (in pixels) between R1 and R2, and between R3 and R4, from the acquired image.

By way of example, in an image acquired in one embodiment, the distance in pixels as measured in the image between R1 and R2 may be found to be 35.7 pixels. It is known that the distance in real space between LED elements R1 and R2 is 15mm.

In the same image, the distance is pixels between R3 and R4 is found to be 37.8 pixels; the distance in real space between LED elements R3 and R4 is also 15mm.

Since the distance in pixels between R1 and R2 is less than the distance in pixels between R3 and R4, it may be determined that a line joining R1 and R2 in real space is further from the camera module 270 than a line joining R3 and R4.

The processing device 300 is configured to calculate a scaling factor for the distance between R1 and R2. Thus, along a line between R1 and R2 in the acquired image, 35.7/15 = 2.38 pixels represent 1 mm.

Similarly, for LED elements R3 and R4, 37.8/15 = 2.52 pixels represent 1mm.

An average of these 2 scaling factors can be determined in order to determine a scaling factor for movement of the blue LED 213: (2.52 + 2.38)/2 = 2.45pixels per mm.

Movement of the blue LED element over a given time period is determined by comparing the position of the blue LED element in one image with its position in a subsequent image. If for example in the time between two consecutive frames (33ms) the blue LED has moved a distance of five pixel columns (ie parallel to the X axis), the absolute distance moved (dx) = 5/2.45 = 2.04mm.

A corresponding calculation can be performed for the movement of the blue LED element across rows of an image (dy) (ie parallel to the Y axis).

The absolute movements (dx and dy) of the blue LED element are then known, with respect to the coordinate system of the camera.

More relevant to measures of movement of the subject is to know the movement when expressed in the coordinates of the subject. In other words, the distance moved (du) in an anterior-posterior direction (parallel to axis V of Figure 7) and the distance moved (dv) in a medial-lateral direction (parallel to axis U of Figure 7).

With reference to Figure 7, the anterior-posterior axis is labelled V. The V axis passes through the blue LED element parallel to lines joining R1 and R3, and R2 and R4, respectively. It is noted that when the housing 201 is attached to the subject, it is attached such that these lines are parallel to the anterior-posterior axis of the subject. Furthermore, the housing is attached such that the anterior-posterior axis of the subject passes through the blue LED element.

A line parallel to the medial-lateral axis is labelled the U axis. The U axis is parallel to lines joining R1 and R2, and R3 and R4, respectively. It will be appreciated that, due to the orientation of the housing 201 with respect to the subject, movements of the blue LED element 213 along the U and V axes corresponds to movement of the subject along the subject's medial-lateral and anterior-posterior axes, respectively.

The values of du and dv may be obtained as follows, with reference to Figure 7.

The value of the angle $\theta$ is first calculated, being the angle between a line connecting R3 and R4, and the X-axis of the coordinate frame of the acquired image.

This can be achieved using Pythagoras' Theorem:

$$\tan \theta = Y1 / X1$$

Thus, movement of the blue LED element in the medial-lateral direction (du), i.e. movement along line the U axis, may be written:

$$du = dx.\cos(\theta) + dy.\sin(\theta)$$

Movement of the blue LED in the anterior-posterior direction (dv), i.e. movement along the V axis, may be written:

$$dv = -dx.\sin(\theta) + dy.\cos(\theta)$$

where dx and dy are absolute displacements along the X, Y axes respectively of the coordinate system of the camera, du and dv are absolute displacements along the U and V axes, respectively, i.e. the coordinate system of the housing 201 attached to the subject. Thus, as discussed above, it is assumed that the housing is attached to the subject such that the axes U, V are parallel to the medial-lateral and anterior-posterior axes, respectively.

Consequently, the subject is not required to stand in such a manner that the U, V axes of the housing are perfectly aligned with the X, Y axes of images acquired by the camera module in order for accurate measurements to be made of movement along the subject's anterior-posterior and medial-lateral axes.

In other words, a person operating the optical balance monitor system is not required to perform a precise alignment of the subject before acquiring data using the system. Intra-observer error in the operation of the system is thereby reduced. Furthermore, since the system is configured to calculate a scaling factor of the distances between LED elements in acquired images, in order to determine a distance of the blue LED element 213 from the camera module 270, the operator is not required to ensure that the camera module 270 is a predetermined distance from the housing 201 before data can be acquired.

In other words, the operator is not required to adjust the position of the camera module 270 when data is acquired corresponding to a subject of a different height from the previous subject.

This has the advantage of increasing an ease with which clinical assessment of a subject may be made. It also reduces a level of training required for a person operating an optical balance monitor according to the second embodiment.

The use of four LED elements to calculate the position of the blue LED element, by means of the simple trigonometry described above, allows compensation for deviations of the plane containing the five LED elements from a horizontal plane. In other words, calibration factors calculated by the system enable movement of the blue LED element 213 to be made regardless of deviations of the array of LED elements 211, 212, 213, 214, 215 from a horizontal plane.

In addition, the use of 4 peripheral calibration elements allows other system features to be included and enables mathematical adjustments to take place which in turn minimise the risk of intra-observer error.

The length of the line joining the left upper and left lower LED elements R1 and R3, when compared with the line joining the right upper and right lower LED elements R2, R4 can be used to calculate the degree of tilt about the anterior-posterior axis V. Similarly, the lines joining the left lower and right lower LED elements R3, R4, and the left upper and right upper LED elements R1, R2, respectively, can be used to calculate the tilt about the medial-lateral axis U.

Furthermore, as described above, a line joining R1 and R2 can be used for example to determine the orientation of the medial-lateral direction, whilst the line joining R1 and R3 can be used to determine the orientation of the anterior-posterior direction.

In the present embodiment of the invention, calibration of pixels of an acquired image along orthogonal directions as described above in terms of movement of the blue LED element 213 is performed for each acquired image. In some embodiments, the calibration of pixels is performed only at the beginning of a trial.

It will be appreciated that the calibration of the positions of LED elements according to the method described above may only be performed when each of the red LED elements R1, R2, R3, R4 is identifiable in an image.

In the event that an LED element moves out of the field of view of the camera module 270 after a trial has started, the system will be unable to determine a location of the element in the acquired image. In these circumstances, systems according to some embodiments of the invention are configured to use the calibration factors most recently determined by the system.

When the LED element that moved out of the field of view returns to the field of view, these systems are configured to once again perform calculations of calibration factors for each acquired image as described above.

It will be appreciated that, in the case that the peripheral LED elements R1, R2, R3, R4 are of a different colour to the central LED element 213, it is easier for the system to determine whether an LED element that moves back into the field of view is a periperhal element or the central LED element 213.

In the event that the peripheral LED elements are themselves of different colours, it will be appreciated that this will further ease the determination of an identity of a peripheral LED element that moves back into the field of view having moved out of the field of view. That is, in some embodiments of the invention it will be easier to determine which LED element corresponds to R1, and which respective LED element corresponds to R2, R3, R4 respectively.

In some embodiments of the invention, it may be useful to know the angle of tilt of the housing 201 about anterior-posterior and medial-lateral axes, respectively. Knowledge of this angle may for example be useful in determining a position of the blue LED more precisely, particularly along the superior-inferior axis of the subject. In other words, determining the distance of the blue LED element 213 below the level of the camera module 270. For the purpose of determining movement of the blue LED element 213, to assess sway movement of the subject, it is typically sufficient to ensure that the array of LED elements 213 is in a plane parallel to the anterior-posterior and medial-lateral axes of the subject, and that the plane of the array also passes through the centre of balance (and therefore the centre of mass (COM)) of the subject.

Nevertheless, knowledge of tilt of the subject during a static posturography trial may be useful to a clinician and may be determined using embodiments of the invention.

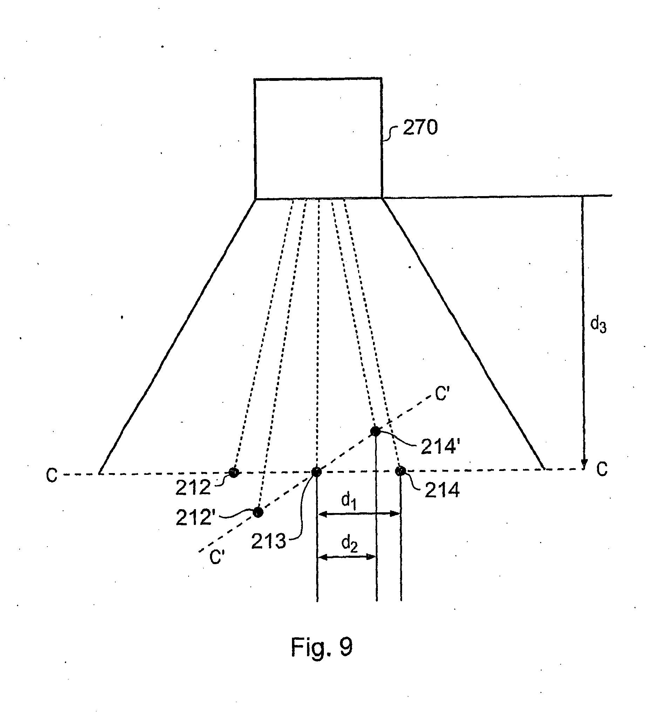

One method of calculating an angle of tilt of the housing 201 between respective sequential acquired images will now be described with reference to Figure 9. It will be appreciated by persons skilled in the art that this method is only one of many methods that may be applied to the assessment and measurement of tilt.

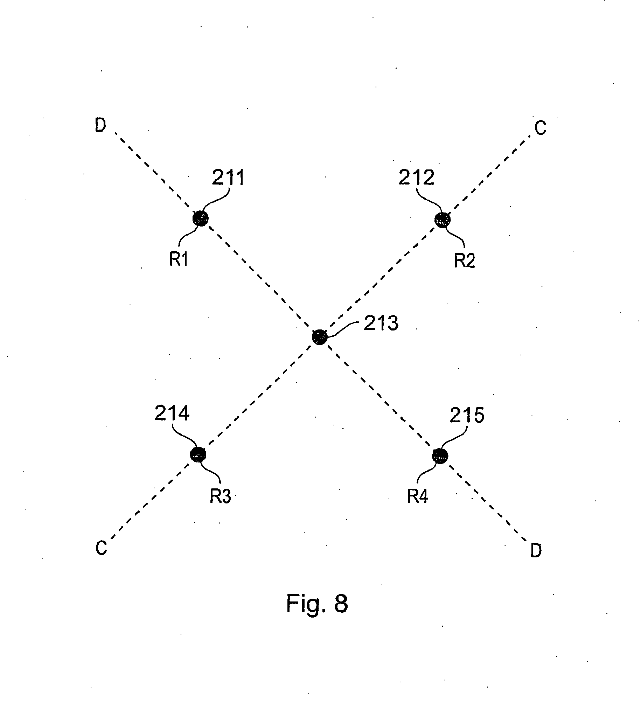

Figure 9 is a cross-sectional schematic diagram of the positions of the image capture device 270 and LED elements 212, 213, 214 when the housing 201 is oriented in a horizontal plane. Line C-C (Figure 8, Figure 9) is arranged to pass through each of the LED elements 212, 213, 214 (i.e. diagonally across the array of LED elements).

The distance between the central blue LED element 213 and red LED element 214 as viewed by the image capture device 270 is indicated as $d_1$ in Figure 9.

If the housing is tilted about an axis along line D-D (Figure 8) that passes through LED elements 211, 213, 215, such that LED elements 212, 214 are displaced to positions labelled 212', 214', respectively in Figure 9, the distance between the central blue LED 213 and red LED element 215 as viewed by the image capture device 270 will now be $d_2$, which is less than $d_1$. Such a tilt about line D-D will be such that the distance $d_3$ of the central blue LED element 213 from the image capture device 270 remains unchanged.

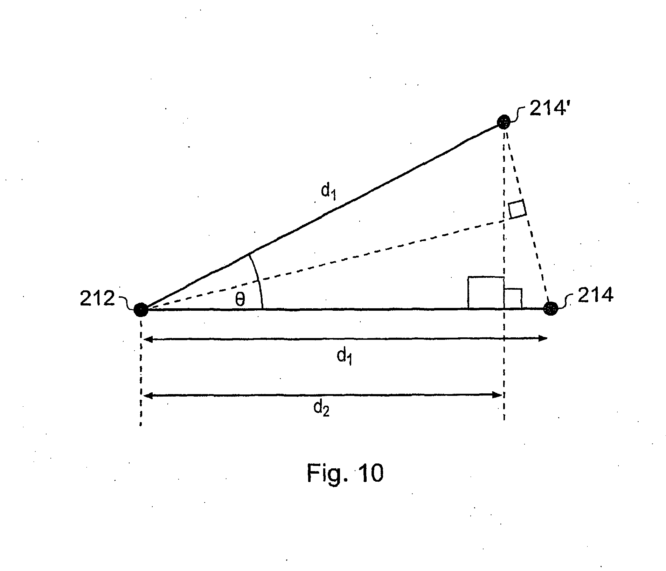

The angle of tilt of the housing 201 about line D-D may then be determined, with reference to Figure 10, from the relationship:

$$\text{Cos } \theta = d_2/d_1$$

It will be appreciated that this calculation, and variations thereof, together with other trigonometric relations, may be used to determine an angle of tilt of the housing 201 about the anterior-posterior axis and the medial-lateral axis by reference to the four peripheral LED elements. In some embodiments further LED elements are provided. In some embodiments the provision of further LED elements enables an angle of tilt about both axes to be determined with increased accuracy.

A series of experiments were performed in order to assess a level of performance of an optical balance monitor system according to the second embodiment of the invention with more complex and expensive prior art systems. It is noted that the system according to the second embodiment that was assembled for the purpose of these trials was constructed using very low cost equipment. For example, a USB2 webcam-type camera device was employed, connected to a laptop computer running the Windows XP™ operating system. The housing 201 was constructed using standard commerically available LED elements.

In the first experiment, the housing 201 was held stationary in a fixed position with respect to the camera module 270 in order to test the precision of the system in measuring displacements of the housing 201 with respect to the camera module 270. In other words, the housing 201 was not attached to a subject, but rather placed on a table positioned beneath the camera module 270.

In subsequent experiments, the housing was connected to the holder 220 of the user attachable portion 200 which was attached to a human subject as shown in Figure 5. A comparison was then made between measurements of displacement of the blue LED 213 of the housing 201 as determined using the optical balance monitor system, and simultaneous measurements of movement of the subject obtained using a commercially available force platform device.

Subjects wearing the user attachable portion 200 of the optical balance monitor system were positioned on a force platform of a force-balance monitor. Movement of the centre of pressure (COP) of the subject was measured using the force-balance monitor during a predetermined period and recorded using a data acquisition device. The predetermined period is hereinafter referred to as the 'trial period'.

Simultaneously, movement of the blue LED element 213 of the optical balance monitor system of the second embodiment of the invention was measured and recorded.

Ten repeated trials were conducted for each subject, first with the subject's eyes open, and then with the subject's eyes closed. Thus, a total of twenty trials were conducted for each subject.

During each trial, the subject stood barefoot, dressed in their usual attire. The feet of each subject were positioned 28cm apart, and the trial period was 30s.

Other positions of the feet of a subject other than a distance of 28cm apart are also useful. Furthermore, other lengths of the trial period are also useful.

After each trial the subject was allowed to rest for 1 minute, and a total of 100 trials were recorded (corresponding to measurements using five subjects).

The force-balance monitor was configured to provide three component signal outputs. The position of the COP was input to a computing device, and stored in a memory of the device.

The anterior-posterior and medial-lateral movements of the user-attachable portion 200 of the optical balance monitor system were plotted against the corresponding components of the measurements of the COP made by the force-balance monitor.

The accuracy in the extraction of position of the COP along the anterior-posterior direction was about 0.1 mm with a variation of from 0.031 to 0.036. Accordingly, linear interpolation was performed on the time-series data to achieve movement data at a controlled sampling rate.

Experiment 1

A first experiment was performed in order to measure the precision with which an optical balance monitor system according to the second embodiment of the invention can measure the position of the housing 201 with respect to the camera module 270.

The user-attachable portion 200 was placed on a table as a support so as to maintain the user-attachable portion 200 in a stationary position with respect to the camera module 270. The camera module 270 was mounted to a tripod support. The user-attachable portion 200 was held in the fixed position for 30s. During this period, the positions of the LED elements were tracked.

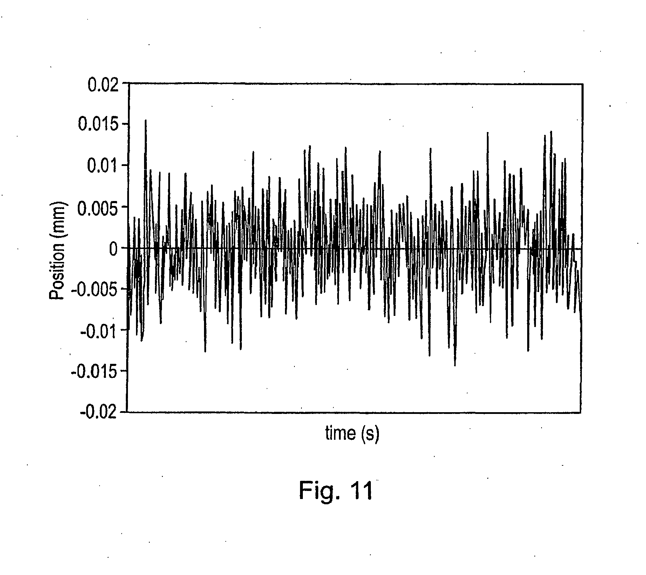

An example of a typical output of such a 'static trial' is presented in Figure 11, with the blue LED element 213 positioned 12cm direclty below the camera module 270. Figure 11 shows raw positional data for the static LED element during the 30s trial period.

With the blue LED element 213 positioned 12cm from the camera module 270, it can be seen that the calculated position of the blue LED element 213 varied over a range of about ±0.015mm.

Larger errors of ±0.03mm occurred when the blue LED element 213 was positioned 240mm from the camera module 270. Smaller errors of ±0.008mm occurred when the blue LED element was positioned 60mm from the camera module 270.

Despite the fact that the optical balance monitor system according to the second embodiment employed a lower resolution camera than those used in most prior art optical motion analysis systems, the precision of the data obtained is comparable with even the most advanced motion analysis systems of which the inventors are aware.

Experiment 2

In a second experiment, the user-attachable portion 200 was attached to a subject as described above, and a static posturogrpahy trial conducted with the subject stood on a force platform device.

A 'sway length' of each subject was determined for each trial using the optical balance monitor system. Simultaneously, the position of the centre of pressure (COP) during the trial was determined using the force platform device.

Furthermore, positions of the COP were also calculated according to an algorithm using the measurements of sway obtained from the optical balance monitor system (see below).

'Sway length' is defined as the summed displacement of the blue LED 213 over a given time period. In the present experiments, this time period is 30s. Other time periods are also useful.

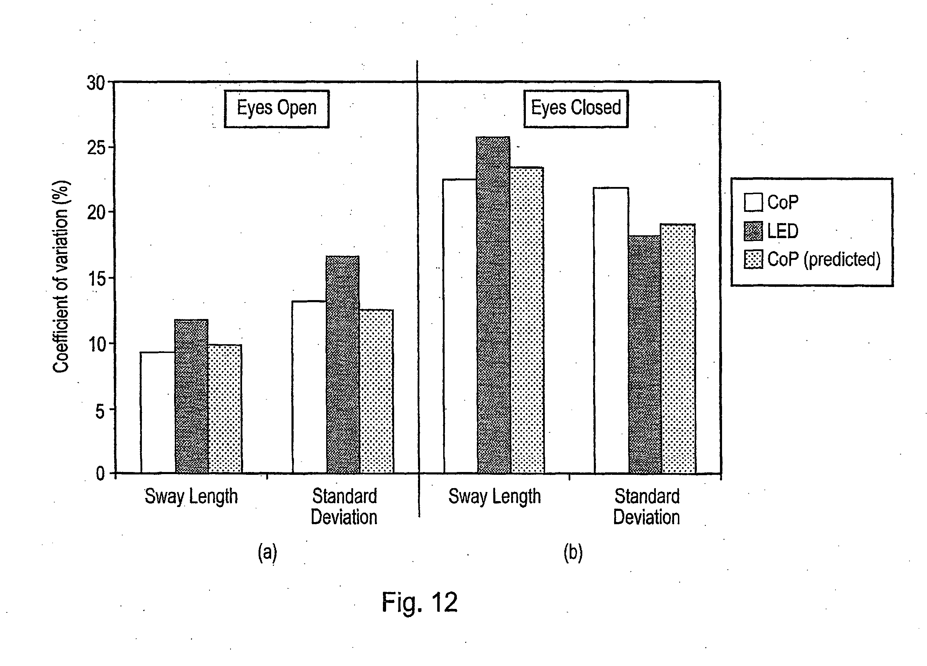

The results of the experiment are shown in Figure 12, for trials performed with the subject's eyes open (Figure 12(a)) and the subject's eyes closed (Figure 12(b)).

Figure 12 shows the values of the coefficient of variation (CoV) for sway variables relating to the sway length of a subject determined as described above. These include sway length as determined by the force platform device (white bars), sway length as determined by the optical balance monitor system (black bars) and sway length based on the values of COP calculated from the COM data obtained using the optical balance monitor system (grey bars).

Also shown in Figure 12 are values of the coefficient of variation for the sway standard deviation (another clinically accepted measure of sway) for the same data.

High coefficients of variation indicate inconsistency in the measurements made, whilst low values indicate consistency in the measurements made. It can be seen that the coefficients of variation of results obtained using the force platform device and the coefficients of variation of results obtained using the optical balance monitor system are similar. Thus, the relatively simple optical balance monitor system according to the present invention achieves comparable levels of consistency when measuring sway to those of more expensive force platform devices.

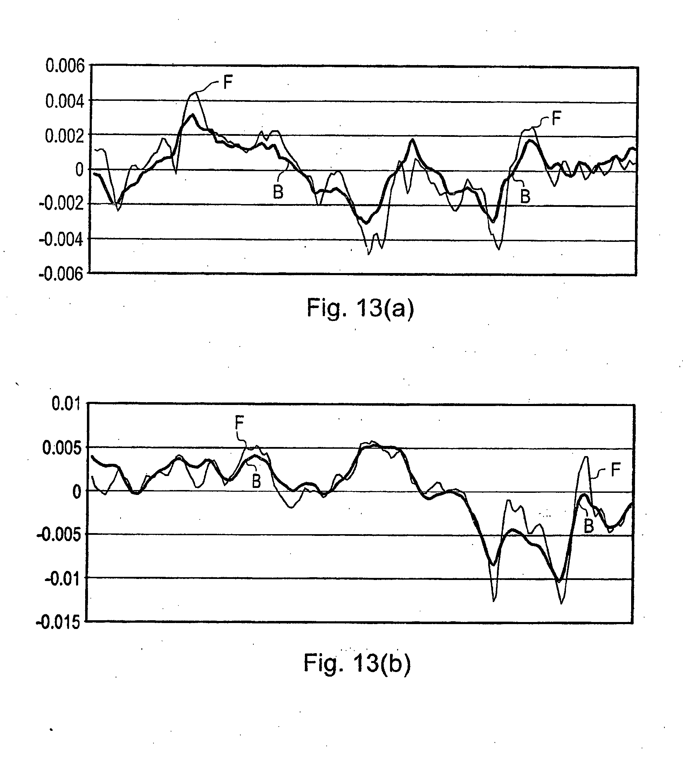

Representative examples of plots of data obtained from the optical balance monitor system and the force platform device are shown in Figure 13. Data obtained by means of the optical balance monitor system, corresponding to movement of the COM, are labelled 'B', whilst data obtained by means of the force platform device, corresponding to movement of the COP, are labelled 'F'. It will be appreciated by persons skilled in the art that exact correspondence between these respective data sets is not to be expected, since COP and COM are different variables.

However, comparison of the signals reveals highly satisfactory levels of synchrony. It can be seen that the range of COP measured using the force-balance monitor is greater than the range of the centre of mass (COM) measured using the optical balance monitor system, as expected. Oscillations measured by the optical balance monitor system are generally in phase with those measured by the force platform, which is also as expected.

Correlation coefficients R between the two sets of time-series data ranged from 0.7 to 0.95, being very strong for the anterior-posterior movements (R = 0.91 to 0.98) and slightly weaker for the medio-lateral movements (R = 0.7-0.9).

The fact that the range of measurements of COP is greater than the range of measurements of COM is in accordance with measurements of COP and COM acquired using far more advanced motion capture systems in static posturography trials. In the medial-lateral direction there is some discrepancy between force platform systems but only when oscillations are low.

The level of agreement between data obtained using an optical balance monitor system according to the second embodiment of the invention is in some ways surprising, given the fact that the system was constructed using especially low cost electronic components. These include a low cost webcam and low cost LED elements. A significant difference in the quality of the data obtained from the system was observed when a USB1 camera was substituted for the USB2 camera of the present invention. The performance of the system was better when a USB2 camera was employed. A further improvement is to be expected when using a USB3 or better camera.

It will be appreciated that the use of more sophisticated image acquisition systems such as cryogenically cooled CCD cameras and other image capture devices would be expected to further improve the performance of the system. More sophisticated data processing systems are also expected to further improve the performance of the system. However, a corresponding increase in the cost of the system is also to be expected.

As discussed above, in addition to providing data relating to movements of a subject's centre of mass, the optical balance monitor system is also provided with an algorithm to predict the position of the COP based on a knowledge of the COM. The COP prediction is based on the inverted pendulum model of Winter et al. (Motor mechanisms of balance during quiet standing, Journal of Electromyography and Kinesiology, Volume 13, Issue 1, February 2003, Pages 49-56, David A. Winter, Aftab E. Patla, Milad Ishac and William H. Gage).

The model describes the relationship between COP and COM according to the equation

$$COP(predicted) = COM - K\sqrt{C\ddot{O}M}$$

where: K=I / Wh, an individual anthropometric variable. I is the total body moment of inertia about the ankles, W is body weight and h is the height of the COM above the ankles. CÖM is the acceleration of the centre of mass, and COM is the position of the centre of mass.

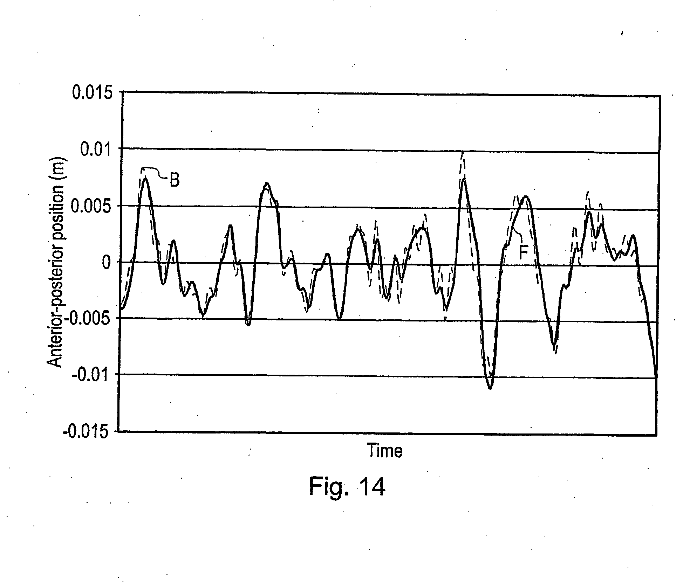

A typical time-series of the predicted position of COP using COM data acquired by the optical balance monitor according to the second embodiment of the invention, and positions of the COP measured using a force platform device, over a 30s time period (for the anterior-posterior direction) is shown in Figure 14.

The COP as measured by the force platform device is shown by the solid line, labelled 'F' whilst the predicted COP using the optical balance monitor device is shown as a dashed line 'B'.

Experiment 3

Sway length and the standard deviation of the sway are both accepted methods for quantifying how much the body is moving during standing. These variables are a way of reducing the displacement vs time data series acquired by the optical balance monitor to a single number, thus allowing comparisions to be made more easily between data sets. The variables are particularly helpful for clinicians involved in subject assessment.

The data sets may be data sets obtained from different patients. Alternatively or in addition the data sets may be obtained from the same subject at different moments in time. For example, data sets obtained at monthly, six-monthly or annual intervals, or any other suitable interval.

Sway length is the summed total displacement of the COM or COP over a given time period (in this case 30s). Sway standard deviation is a measure of how much the COM or COP is oscillating about its mean position.

The visual system of the human balance system is important in maintaining balance, and it is therefore to be expected that the sway variables of a subject will be lower in the eyes-open condition compared with the eyes-closed condition.

The ability of a system to distinguish between the eyes-open and eyes-closed conditions provides an indication as to how effective that system is in detecting deterioration/improvement in balance.

Sway variables were calculated using the optical balance monitor system and the force platform device for a series of trials (eyes-open and eyes-closed). The data was acquired in the course of measurments performed with five subjects during the course of ten trials with their eyes open and ten trials with their eyes closed was used.

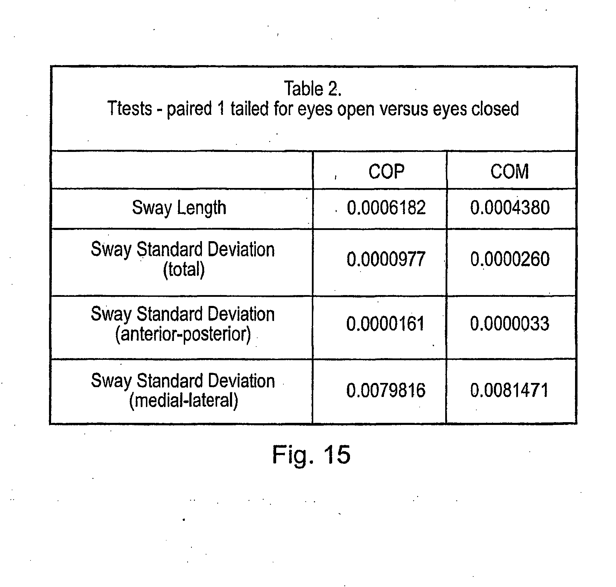

Paired two-tailed t-tests (Figure 15) were obtained to highlight the differences between the two techniques.

Sway length (Figure 15) was found to be on average 1.58 times greater using the force platform device compared with 1.76 for the optical balance monitor. Similarly, sway SD was on average 1.85 greater as measured using the force platform device compared with 1.95 as measured using the optical balance monitor system. This resulted in slightly higher significance (p= 0.00088) for the optical system compared with p = 0.00124 for the force platform device. In addition, sway standard deviation was 0.002 using the force platform device, and 0.00005 using the optical system.

These results indicate that the optical blance monitor system is in fact slightly better than the force platform device in distinguishing between the eyes-open and eyes-closed conditions.

Embodiments of the present invention may be used for:

> 1. Rehabilitation (stroke and head injuries) including elements of real-time games to make rehabilitation exercises less boring.
>
> 2. Training - to provide sport science-based fitness protocols (e.g. beep testing), games to make fitness training less boring and games to specifically train different types of fitness (e.g. speed, endurance, power, anaerobic) or muscle groups.
>
> 3. Training aid for precision sports e.g. golf, darts, archery, snooker, pool, shooting etc.

In some embodiments of the invention, the array of LED elements of the housing 201 are not present. Rather, a feature such as a symbol, shape or other indice is provided on the housing for viewing by the camera module 270. The feature may include an array of dots or other shapes of a single colour (eg white) on a black background. Alternatively the feature may include an array of coloured shapes. In some embodiments the feature includes an array of four red dots and a central blue dot, the five dots being arranged in the form of the 'five' face of a gaming die.

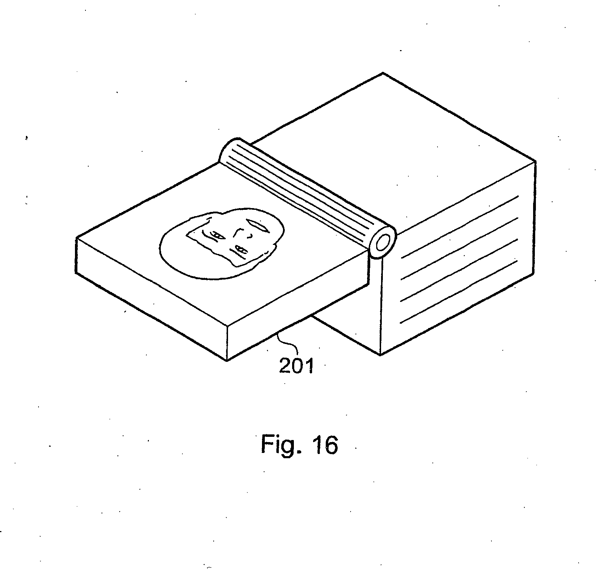

In some embodiments, the feature includes a 2D shape such as a pentagon, a hexagon, or any other suitable polygonal shape. In some embodiments, the feature includes a 3D shape such as a cuboid, a tetrahedron, or any other suitable polyhedral shape. In some embodiments, the feature is illuminated; in some embodiments the feature is illuminated from behind the feature, in other embodiments the feature is illuminated from in front of the feature, with respect to the camera module. In some embodiments the feature is an image recognisable by a user and configured to provide comfort or entertainment, such as a face (Figure 16).

In some embodiments of the invention, the illumination source is co-located with the camera module 270. In some embodiments, the feature includes reflective elements arranged to reflect light to the camera module 270. In some of these embodiments the reflective elements are arranged to reflect infra-red (IR) radiation towards the camera. In some embodiments an IR light source is provided. The IR light source may be co-located with the camera module 270.

It will be appreciated that some embodiments of the invention may be useful in dynamic posturography studies, in addition to or instead of static posturography trials.

For example, in some embodiments the system is configured to measure sway of a subject walking on a treadmill. According to some of these embodiments, a subject wearing a user-attachable portion 200 is positioned on a moving portion of a treadmill. A camera module 270 is mounted in fixed relationship to a stationary portion of the treadmill and is arranged to capture images of the LED elements of the housing 201, as described above. Sway data is then acquired as the subject walks on the treadmill.

It will be appreciated that an increased risk exists that one or more LED elements will move out of the field of view of the camera module 270 during the course of a dynamic posturography trial. The processing device 300 according to some embodiments of the invention is therefore configured to compensate for such events, as described above.

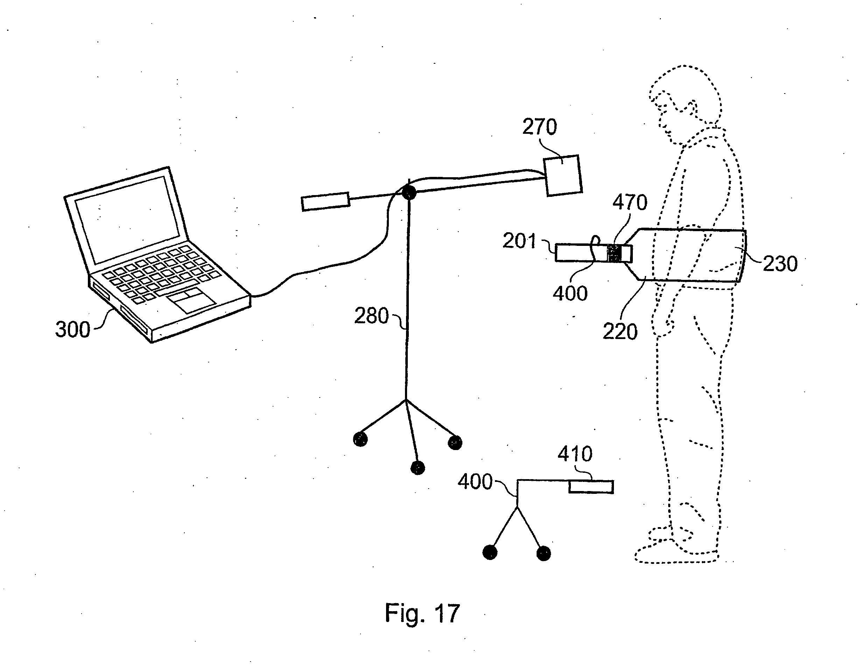

According to a third embodiment of the invention, an independent tilt monitor 400 is provided (Figure 17). Reference signs in Figure 17 correspond with those used in relation to the second embodiment of the invention, for features in common with the second embodiment.

The tilt monitor 400 comprises a camera module 470 attachable to the subject under examination, and an array of tilt monitor LED elements 410. The tilt monitor 400 is configured to acquire images of the tilt monitor LED elements 410 using the camera module 470. A processing module is then configured to determine a tilt angle of the camera module (and therefore of the user) with respect to a reference axis, from the acquired image.

According to the third embodiment the tilt angle is determined by comparing an acquired image with a reference image. In some embodiments the reference image is acquired with the subject in a particular position. In alternative embodiments, the processing module is configured to determine a tilt angle directly from a position of the LED elements 410.

In some embodiments of the invention, the array of LED elements 410 is replaced by a single light emitting device. In some embodiments of the invention, a different reference feature is used, such as a symbol marked on an article, or any suitable reference feature provided by a user of the system. In some embodiments the reference feature is any suitable feature in an image, such as a foot of the subject, a portion of a surface on which the user is standing, or any other suitable feature.

According to variations of the third embodiment, the angle of tilt is determined by analysing the relative positions of the LED elements 410 with respect to one another. In other words, the system exploits the feature that distortion of the appearance of the array of elements 410 in an acquired image occurs as the camera module 470 is tilted.

It will be appreciated that attachment of a camera module 470 to the subject, and fixing the LED elements 410 in relation to ground on which the user stands amplifies movement of the LED elements 410 with respect to boundaries of an acquired image when a user tilts, compared with a configuration in which the camera is fixed in relation to the ground, and the LED elements 410 are attached to the subject.

**Claims**

1. A system for monitoring a person's balance, the system comprising:

   i) a unit (201) bearing at least one indicium (211,212,213,214,215), wherein the at least one indicium (211,212,213,214,215) is/are configured to provide at least two spatially resolved reference positions; and
   ii) an image capture device (207) having a single field of view;

   wherein the at least one indicium (211,212,213,214,215) have a fixed predetermined spatial configuration on the unit (201);
   wherein either the unit (201) or the image capture device (207) is provided with a means for attachment (230) to a person such that the unit (201) or device (207) is locatable generally at a centre of balance of a subject, the system being configured to record movement, in the single field of view of the image capture device, of the at least one indicia (211,212,213,214,215) with reference to the subject's centre of balance and calculate the distances of the at least two spatially resolved reference positions of the unit from the image capture device so as to obtain an objective measure of balance.

2. A system as claimed in claim 1 further comprising a computer (300) in communication with the image capture device (207).

3. A system as claimed in claim 1 or 2 wherein the unit (201) is arranged to bear at least two light emitting or reflecting indicia (211,212,213,214,215), the indicia (211,212,213,214,215) having a fixed predetermined spatial configuration on the unit (201).

4. A system as claimed in claim 3 wherein at least one of the indicia (211,212,213,214,215) is arranged to emit/reflect light of a different characteristic from the other indicium/indicia (211,212,213,214,215).

5. A system as claimed in claim 3 or claim 4 wherein the light emitting/reflecting indicia (211, 212, 213, 214,215) are configured to project images having different shapes.

6. A system as claimed in any one of claims 3 to 5 wherein two of the indicia (211,212,213,214,215) are arranged to emit/reflect light of a different respective colour from one another, the indicia (211, 212, 213, 214,215) having a fixed predetermined spatial configuration on the unit (201), and the colors being selected to correspond with at least two channels of an image capture device (207), optionally the unit (201) having three light emitting/reflecting indicia, respectively emitting/reflecting red, green and blue light to correspond with red, green and blue channels of an image capture device (207).

7. A system as claimed in any one of claims 3 to 6 wherein the unit (201) comprises three light emitting /reflecting indicia having a substantially linear arrangement.

8. A system as claimed in claim 7 wherein a central light emitting/reflecting indicium (213) is locatable generally at the centre of balance of a subject.

9. A system as claimed in any one of claims 3 to 8, comprising five light emitting/reflecting indicia (211, 212,213,214,215) arranged such that a central light emitting/reflecting indicium (213) is surrounded by four light emitting/reflecting indicia (211, 212, 214, 215) arranged so as to define corners of a notional quadrilateral enclosing the central light emitting/reflecting indicium (213).

10. A system as claimed in claim 9 wherein the four light emitting/reflecting indicia (211,212,214,215) arranged to define corners are different in colour to the central light emitting/reflecting indicium (213), optionally wherein at least one of the four light emitting/reflecting indicia (211,212,214,215) arranged to define corners is of a different colour to the other three indicia arranged to define corners.

11. A system as claimed in any one of claims 3 to 10 wherein the unit (201) comprises at least one light reflective indicium (211,212,213,214,215) and the system further comprises a light source arranged to illuminate the at least one light-reflective indicium, wherein optionally the light source is co-located with the image capture device (207).

12. A system as claimed in claim 11 wherein the light source is an infra-red (IR) light source, said at least one light-reflective indicium being arranged to reflect IR light.

13. A system as claimed in any preceding claim wherein the image capture device (207) is configured so as to capture images in which substantially all background detail is obscured, leaving only images of the at least one indicium.

14. A system as claimed in any preceding claim further configured to determine a sway variable of the subject based on the measured movement of the unit (201).

15. A method of monitoring a person's balance, the method comprising the steps of:

   i) providing a unit (201) bearing at least two light emitting/reflecting indicia (211, 212, 213, 214, 215), the at least two light emitting/reflecting indicia (211,212,213,214,215)having a fixed predetermined spatial configuration on the unit (201);
   ii) providing an image capture device (207);
   iii) attaching one of either the unit (201) or the image capture device (207)_to a person, preferably at or near the person's centre of balance;
   iv) mounting the remaining one of either the unit (201) or the image capture device (207) so as to face the person;
   v) recording the movement of the light emitting/reflecting indicia (211,212,213,214,215) with the image capture device while the person performs a range of movements;
   vi) using a computer (300) to determine movement of the light emitting/reflecting indicia (211,212,213,214,215) with reference to the person's centre of balance and calculate the distances of the at least two spatially resolved reference positions of the unit from the image capture device so as to obtain an objective measure of balance.

**Patentansprüche**

1. System zum Überwachen des Gleichgewichtes einer Person, wobei das System umfasst:

i) eine Einheit (201), die mindestens ein Kennzeichen (211, 212, 213, 214, 215) trägt, wobei das mindestens eine Kennzeichen (211, 212, 213, 214, 215) konfiguriert ist, um mindestens zwei räumlich aufgelöste Referenzstandorte zur Verfügung zu stellen; und
ii) eine Bilderfassungsvorrichtung (207), die über ein einzelnes Bildfeld verfügt;

wobei das mindestens eine Kennzeichen (211, 213, 213, 214, 215) über eine feste vorbestimmte räumliche Konfiguration auf der Einheit (201) verfügt; wobei entweder die Einheit (201) oder die Bilderfassungsvorrichtung (207) mit einem Mittel zur Befestigung (230) an eine Person ausgestattet ist, so dass die Einheit (201) oder die Vorrichtung (207) im Allgemeinen bei einem Schwerpunkt eines Subjektes lokalisierbar ist, wobei das System konfiguriert ist, um eine Bewegung, in dem einzelnen Bildfeld der Bilderfassungsvorrichtung, des mindestens einen Kennzeichens (211, 212, 213, 214, 215) bezüglich des Schwerpunktes einer Person aufzuzeichnen und die Entfernungen der mindestens zwei räumlich aufgelösten Referenzstandorte der Einheit von der Bilderfassungsvorrichtung zu berechnen, um so ein objektives Maß eines Gleichgewichtes zu erhalten.

2. System gemäß Anspruch 1, das weiterhin einen Computer (300) in Kommunikation mit der Bilderfassungsvorrichtung (207) umfasst.

3. System gemäß Anspruch 1 oder 2, wobei die Einheit (201) angeordnet ist, um mindestens zwei lichtemittierende oder -reflektierende Kennzeichen (211, 212, 213, 214, 215) zu tragen, wobei die Kennzeichen (211, 212, 213, 214, 215) über eine feste vorbestimmte räumliche Konfiguration auf der Einheit (201) verfügen.

4. System gemäß Anspruch 3, wobei mindestens eines der Kennzeichen (211, 212, 213, 214, 215) angeordnet ist, um Licht mit einem anderen Merkmal als das/die andere(n) Kennzeichen (211, 212, 213, 214, 215) zu emittieren/reflektieren.

5. System gemäß Anspruch 3 oder Anspruch 4, wobei die lichtemittierenden/- reflektierenden Kennzeichen (211, 212, 213, 214, 215) konfiguriert sind, um Bilder mit unterschiedlichen Formen zu projizieren.

6. System gemäß einem der Ansprüche 3 bis 5, wobei zwei der Kennzeichen (211, 212, 213, 214, 215) angeordnet sind, um Licht von jeweils unterschiedlicher Farbe zu emittieren/reflektieren, wobei die Kennzeichen (211, 212, 213, 214, 215) über eine feste vorbestimmte räumliche Konfiguration auf der Einheit (201) verfügen und die Farben ausgewählt werden, um mit mindestens zwei Kanälen einer Bilderfassungsvorrichtung (207) übereinzustimmen, wobei die Einheit (201) optional über drei lichtemittierende/-reflektierende Kennzeichen verfügt, die jeweils rotes, grünes und blaues Licht emittieren/reflektieren, um mit einem roten, grünen und blauen Kanal einer Bilderfassungsvorrichtung (207) übereinzustimmen.

7. System gemäß einem der Ansprüche 3 bis 6, wobei die Einheit (201) über drei lichtemittierende/-reflektierende Kennzeichen verfügt, die über eine im Wesentlichen lineare Anordnung verfügen.

8. System gemäß Anspruch 7, wobei ein zentrales lichtemittierendes/- reflektierendes Kennzeichen (213) im Allgemeinen bei dem Schwerpunkt eines Subjektes lokalisierbar ist.

9. System gemäß einem der Ansprüche 3 bis 8, das fünf lichtemittierende/- reflektierende Kennzeichen (211, 212, 213, 214, 215) umfasst, die so angeordnet sind, dass ein zentrales lichtemittierendes/-reflektierendes Kennzeichen (213) von vier lichtemittierenden/-reflektierenden Kennzeichen (211, 212, 214, 215) umgeben wird, die so angeordnet sind, dass sie Ecken eines fiktiven Vierecks, das das zentrale lichtemittierende/-reflektierende Kennzeichen (213) enthält, definieren.

10. System gemäß Anspruch 9, wobei die vier lichtemittierenden/-reflektierenden Kennzeichen (211, 212, 214, 215), die angeordnet sind, um Ecken zu definieren, sich farblich von dem zentralen lichtemittierenden/-reflektierenden Kennzeichen (213) unterscheiden, wobei sich eines der vier lichtemittierenden Kennzeichen (211, 212, 214, 215), die angeordnet sind, um Ecken zu definieren, optional farblich von den anderen drei Kennzeichen, die angeordnet sind, um Ecken zu definieren, unterscheidet.

11. System gemäß einem der Ansprüche 3 bis 10, wobei die Einheit (201) mindestens eine lichtemittierendes/-reflektierendes Kennzeichen (211, 213, 213, 214, 215) umfasst und das System weiterhin umfasst: eine Lichtquelle, die angeordnet ist, um das mindestens eine lichtemittierende/-reflektierende Kennzeichen zu beleuchten, wobei die Lichtquelle optional zusammen mit der Bilderfassungsvorrichtung (207) angeordnet ist.

12. System gemäß Anspruch 11, wobei die Lichtquelle eine Infrarot (IR)-Lichtquelle ist, wobei das mindestens eine lichtreflektierende Kennzeichen angeordnet ist, um IR-Licht zu reflektieren.

13. System gemäß einem der vorangehenden Ansprüche, wobei die Bilderfassungsvorrichtung (207) konfiguriert ist, um Bilder zu erfassen, in denen im Wesentlichen alle Hintergrunddetails verdeckt sind, wodurch nur Bilder des mindestens einen Kennzeichens zurückgelassen werden.

14. System gemäß einem der vorangehenden Ansprüche, das weiterhin konfiguriert ist, um eine Schlingervariable des Subjektes basierend auf der gemessenen Bewegung der Einheit (201) zu bestimmen.

15. Verfahren eines Überwachens des Gleichgewichtes einer Person, wobei das Verfahren die folgenden Schritte umfasst:

i) Bereitstellen einer Einheit (201), die mindestens zwei lichtemittierende/- reflektierende Kennzeichen (211, 212, 213, 214, 215) trägt, wobei die mindestens zwei lichtemittierenden/-reflektierenden Kennzeichen (211, 212, 213, 214, 215) über eine feste vorbestimmte räumliche Konfiguration auf der Einheit (201) verfügen;

ii) Bereitstellen einer Bilderfassungsvorrichtung (207);

iii) Befestigen entweder der Einheit (201) oder der Bilderfassungsvorrichtung (207) an eine Person, vorzugsweise bei oder nahe dem Schwerpunkt der Person;

iv) Montieren der verbleibenden entweder Einheit (201) oder Bilderfassungsvorrichtung (207), so dass die Einheit oder Vorrichtung der Person gegenüberliegen;

v) Aufzeichnen der Bewegung der lichtemittierenden/-reflektierenden Kennzeichen (211, 212, 213, 214, 215) mit der Bilderfassungsvorrichtung, während die Person einen Bereich von Bewegungen durchführt;

vi) Verwenden eines Computers (300), um eine Bewegung der lichtemittierenden/-reflektierenden Kennzeichen (211, 212, 213, 214, 215) bezüglich des Schwerpunktes einer Person zu bestimmen und die Abstände der mindestens zwei räumlich aufgelösten Referenzstandorte der Einheit von der Bilderfassungsvorrichtung zu berechnen, um so ein objektives Maß eines Gleichgewichtes zu erhalten.

**Revendications**

1. Système pour contrôler l'équilibre d'une personne, le système comprenant:

i) une unité (201) supportant au moins un indice (211, 212, 213, 214, 215), dans lequel le au moins un indice (211, 212, 213, 214, 215) est configuré pour fournir au moins deux positions de référence spatialement déterminées; et
ii) un dispositif de capture d'image (207) ayant un seul champ de vision;

dans lequel le au moins un indice (211, 212, 213, 214, 215) a une configuration spatiale prédéterminée fixe sur l'unité (201);
dans lequel l'unité (201) ou le dispositif de capture d'image (207) est prévu(e) avec des moyens pour se fixer (230) sur une personne de sorte que l'unité (201) ou le dispositif (207) peut être généralement positionné(e) au niveau d'un centre d'équilibre d'un patient, le système étant configuré pour enregistrer le déplacement, dans l'unique champ de vision du dispositif de capture d'image, du au moins un indice (211, 212, 213, 214, 215) en référence au centre d'équilibre du patient et calculer les distances des au moins deux positions de référence spatialement déterminées de l'unité à partir du dispositif de capture d'image afin d'obtenir une mesure d'équilibre objective.

2. Système selon la revendication 1, comprenant en outre un ordinateur (300) en communication avec le dispositif de capture d'image (207).

3. Système selon la revendication 1 ou 2, dans lequel l'unité (201) est agencée afin de supporter au moins deux indices d'émission ou de réflexion de lumière (211, 212, 213, 214, 215), les indices (211, 212, 213, 214, 215) ayant une configuration spatiale prédéterminée fixe sur l'unité (201).

4. Système selon la revendication 3, dans lequel au moins l'un des indices (211, 212, 213, 214, 215) est agencé pour émettre / refléter la lumière d'une caractéristique différente de l'autre (des autres) indice / indices (211, 212, 213, 214, 215).

5. Système selon la revendication 3 ou la revendication 4, dans lequel les indices d'émission / réflexion de lumière (211, 212, 213, 214, 215) sont configurés pour projeter des images ayant des formes différentes.

6. Système selon l'une quelconque des revendications 3 à 5, dans lequel deux des indices (211, 212, 213, 214, 215) sont agencés pour émettre / refléter de la lumière d'une couleur respective différente d'une autre, les indices (211, 212, 213, 214, 215) ayant une configuration spatiale prédéterminée fixe sur l'unité (201) et les couleurs étant sélectionnées pour correspondre à au moins deux canaux d'un dispositif de capture d'image (207), facultativement, l'unité (201) ayant trois indices d'émission / réflexion de lumière, émettant / reflétant respectivement de la lumière rouge, verte et bleue pour correspondre aux canaux rouge, vert et bleu d'un dispositif de capture d'image (207).

7. Système selon l'une quelconque des revendications 3 à 6, dans lequel l'unité (201) comprend trois indices d'émission / réflexion de lumière ayant un agencement sensiblement linéaire.

8. Système selon la revendication 7, dans lequel un indice d'émission / réflexion de lumière central (213) peut généralement être positionné au niveau du centre d'équilibre d'un patient.

9. Système selon l'une quelconque des revendications 3 à 8, comprenant cinq indices d'émission / réflexion de lumière (211, 212, 213, 214, 215) agencés de sorte qu'un indice d'émission / réflexion de lumière central (213) est entouré par quatre indices d'émission / réflexion de lumière (211, 212, 214, 215) agencés afin de définir des coins d'un quadrilatère théorique enfermant l'indice d'émission / réflexion de lumière central (213).

10. Système selon la revendication 9, dans lequel les quatre indices d'émission / réflexion de lumière (211, 212, 214, 215) agencés afin de définir des coins ont une couleur différente de l'indice d'émission / réflexion de lumière central (213), facultativement dans lequel au moins l'un des quatre indices d'émission / réflexion de lumière (211, 212, 214, 215) agencés pour définir des coins a une couleur différente des trois autres indices agencés pour définir des coins.

11. Système selon l'une quelconque des revendications 3 à 10, dans lequel l'unité (201) comprend au moins un indice de réflexion de lumière (211, 212, 213, 214, 215) et le système comprend en outre une source de lumière agencée pour éclairer le au moins un indice de réflexion de lumière, dans lequel, facultativement, la source de lumière est positionnée conjointement avec le dispositif de capture d'image (207).

12. Système selon la revendication 11, dans lequel la source de lumière est une source de lumière infrarouge (IR), ledit au moins un indice de réflexion de lumière étant agencé pour refléter la lumière IR.

13. Système selon l'une quelconque des revendications précédentes, dans lequel le dispositif de capture d'image (207) est configuré afin de capturer des images dans lesquelles sensiblement la totalité des détails d'arrière-plan est obscurcie, laissant uniquement des images du au moins un indice.

14. Système selon l'une quelconque des revendications précédentes, configuré en outre pour déterminer une variable de balancement du patient en fonction du mouvement mesuré de l'unité (201).

15. Procédé pour contrôler l'équilibre d'une personne, le procédé comprenant les étapes consistant à:

i) prévoir une unité (201) supportant au moins deux indices d'émission / réflexion de lumière (211, 212, 213, 214, 215), les au moins deux indices d'émission / réflexion de lumière (211, 212, 213, 214, 215) ayant une configuration spatiale prédéterminée fixe sur l'unité (201);
ii) prévoir un dispositif de capture d'image (207);
iii) fixer l'unité (201) ou le dispositif de capture d'image (207) sur une personne, de préférence au niveau de ou à proximité du centre d'équilibre de la personne;
iv) monter le reste de l'unité (201) ou du dispositif de capture d'image (207) afin de faire face à la personne;
v) enregistrer le mouvement des indices d'émission / réflexion de lumière (211, 212, 213, 214, 215) avec le dispositif de capture d'image alors que la personne réalise une série de mouvements;
vi) utiliser un ordinateur (300) pour déterminer le mouvement des indices d'émission / réflexion de lumière (211, 212, 213, 214, 215) en référence au centre d'équilibre de la personne et calculer les distances des au moins deux positions de référence spatialement déterminées de l'unité à partir du dispositif de capture d'image, afin d'obtenir une mesure d'équilibre objective.

Fig. 1

Fig. 3

Fig. 2

18

Fig. 4

Fig. 5

Fig. 6A

Fig. 6B

Fig. 6C

Fig. 7

D
211
R1

C
212
R2

213

214
R3

215
R4

C

D

Fig. 8

Fig. 9

Fig. 10

Fig. 11

Fig. 12

Fig. 13(a)

Fig. 13(b)

Fig. 14

| Table 2.<br>Ttests - paired 1 tailed for eyes open versus eyes closed | | |
| --- | --- | --- |
| | COP | COM |
| Sway Length | 0.0006182 | 0.0004380 |
| Sway Standard Deviation<br>(total) | 0.0000977 | 0.0000260 |
| Sway Standard Deviation<br>(anterior-posterior) | 0.0000161 | 0.0000033 |
| Sway Standard Deviation<br>(medial-lateral) | 0.0079816 | 0.0081471 |

Fig. 15

Fig. 16

Fig. 17

EP 2 023 816 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4631676 A
- DE 4328371
- US 6324296 B
- US 20050105772 A, Voronka
- WO 9944502 A, Claussen

### Non-patent literature cited in the description

- **WINTER ; DAVID A. WINTER ; AFTAB E. PATLA ; MILAD ISHAC ; WILLIAM H. GAGE et al.** Motor mechanisms of balance during quiet standing. *Journal of Electromyography and Kinesiolog,* February 2003, vol. 13 (1), 49-56